# EUROPEAN PATENT SPECIFICATION

(11) **EP 1 922 097 B1**
(45) Date of publication and mention of the grant of the patent: **20.10.2010**
(21) Application number: 06791735.1
(22) Date of filing: 30.08.2006
(51) Int. Cl.: A61M 1/36, B01D 27/06

(54) **METHOD AND APPARATUS FOR THE REMOVAL OF IMMUNE CELLS**
VERFAHREN UND GERÄT ZUR ENTFERNUNG VON IMMUNZELLEN
PROCÉDÉ ET APPAREIL DE PRÉLÈVEMENT DES CELLULES IMMUNITAIRES

(30) Priority: 31.08.2005 US 713527 P; 10.04.2006 US 744571 P
(43) Date of publication of application: 21.05.2008
(73) Proprietor: Gambro Lundia AB, 22 643 Lund (SE)
(72) Inventor: DEPPISCH, Reinhold, 72379 Hechingen (DE); WITTNER, Bernd, 72379 Hechingen (DE); KOCH, Sylvia, 72458 Albstadt (DE)
(74) Representative: Hornung, Veronika Margot
(86) International application number: PCT/EP2006/008482
(87) International publication number: WO 2007/025735

(56) References cited:
- EP-A1- 0 502 213
- WO-A-93/04763
- WO-A-98/08557
- JP-A- 2005 144 330
- US-A- 4 361 483
- US-A- 5 632 894
- US-A1- 2003 057 147

## Description

### FIELD OF THE INVENTION

This invention relates to a method and apparatus for the removal of immune cells which may play a role in contributing to a disease state.

### BACKGROUND

Sepsis and systemic inflammatory response syndrome (SIRS) is the leading cause of death in intensive care units, with mortality rates reported at being between 30-70%. The costs in the United States alone for treatment of sepsis have been calculated to be around 16.7 billion per year.

Sepsis and SIRS represent a wide spectrum of clinical symptoms. Originally, the diagnosis of sepsis required confirmation of bacterial growth in blood cultures as well as the presence of two or more of the following symptoms: hypothermia (<36° C), hyperthermia (>38° C), tachycardia (>90 beats/min), tachypnea (>20 breaths/min or P_{CO2}<32 mmHg) and leukocytopenia (<4 x 10⁹ cells/L) or leukocytosis (>12 x 10⁹ cells/L).

Currently, the diagnosis of SIRS is used when the patient has two or more of the above symptoms but no evidence of bacteria can be found in the blood, which includes at least 50% of patients having the above-described symptoms. SIRS often progresses to severe cases of sepsis at which time organ failure and often death occurs.

Sepsis results from the body's systemic over-response to infection. During the onset of sepsis, the inflammatory system becomes hyperactive, involving both cellular and humoral defense mechanisms. Endothelial cells and epithelial cells, as well as neutrophils, macrophages and lymphocytes produce pro-inflammatory mediators, especially tumor necrosis factor-α (TNF-α), interleukins IL-6, IL-1 and IL-8. Simultaneously, production of acute phase proteins such as C-reactive protein occurs and humoral defense mechanisms such as the complement system are activated, resulting in production of pro-inflamatory mediators including C5a. C5a enhances cytokine and chemokine production.

The above-described mediators are produced early in the onset of sepsis and reflect the overactive status of the inflammatory response. Phagocytic cells such as granulocytes respond to many of these pro-inflammatory mediators by releasing granular enzymes and producing reactive oxygen species (ROS) such as hydrogen peroxide (H₂O₂) which is a crucial product for killing bacteria. However, H₂O₂ also is capable of causing tissue damage, which ultimately leads to increased vascular permeability and organ injury. In the later stages of sepsis, anti-inflammatory mediators are produced (such as IL-10, TGF-β and IL-13) which stop the production of many pro-inflammatory mediators. In this phase, the functions of neutrophils are suppressed, which leads to a hyporeactive host defense system and immunoparalysis.

In summary, the above cycle of events which ultimately ends in sepsis can be broken down into three phases. The first phase is begun by introduction of substances into the body such as bacteria and/or by-products of bacteria which activates the body's immune cells to produce and secrete cytokines. The second phase is begun by the cytokines themselves, which activate other immune cells to produce even more cytokines. The activated immune cells themselves begin phase three, which leads to a hyperreactive defense system.

There are many pharmaceutical as well as extracorporeal treatments which exist to treat one or more aspects of the three phases above. Pharmaceutical treatments such as antibiotics are used to treat the initial activator (first phase) of the immune system (bacteria). Other pharmaceuticals such as anti-inflammatories are used to block the pro-inflammatory response of cytokines and to stabilize the immune system to treat the second phase. Extracorporeal blood purification such as plasmapheresis to remove accumulating cytokines in the plasma is also used. However, none of these treatments alone or in combination have produced better clinical outcomes.

Activated white blood cells are also thought to play an important role in other disease states such as organ failure following transplantation. This may be because the newly transplanted organ has restricted blood flow. During reperfusion of the newly transplanted organ with blood, activated white blood cells may massively infiltrate the organ and mediate tissue damage, ultimately causing organ failure.

Activated white blood cells may also play a role in acute renal failure. In a study done by Rab et al., mononuclear cell infiltrates were found in kidneys that had suffered ischemia followed by reperfusion. This group found that if white blood cell infiltration into the ischemic kidneys was reduced, tubular necrosis was also reduced.

Activated white blood cells may also play a role in the development of other diseases, such as rheumatoid arthritis, lupus erythematosus, collagenoses, and ulcerative colitis.

Activated cells are defined as peripheral white blood cells which are capable of exerting damage on an organ when left in circulation.

White blood cells, whether activated or not, also cause an estimated 90% of all adverse reactions from blood transfusions whether the transfusion recipient receives whole blood or separated blood components.

Therefore the present invention is directed towards reducing complications caused by white blood cells by removing them from whole blood or blood components.

WO 93/04763 A1 discloses a leukocyte depletion filter assembly comprising a housing having an inlet and an outlet, a vent and a liquid flow path between the inlet and the outlet and a degassing element communicating with the vent; and a porous medium positioned in the housing across the fluid path, the surface of the porous medium having been modified by exposure to a gas plasma stream.

US 5,632,894 A discloses a blood filter with a filtration element surrounding at least a portion of the axis defined by the side walls. The filtration element comprises a cylindrical pleated filter member. The reference does not mention the material of the filter member.

US 2003/0057147 A1 discloses a filtration device including a filtration assembly disposed within a housing. The filtration assembly includes a central core projecting into the housing and surrounded by a filter material. The central core is a longitudinal tube having pores or holes in its side wall. Filter materials mentioned are needle punched fabric, micro-glass, PVA/PAN copolymer acrylic pulp combinations, polyester screen, PBT polyester screen, nylon, acrylics, and cellulose acetate.

EP 0 502 213 A1 discloses a method of removing leukocytes from a leukocyte-containing blood preparation which uses a microfilter element made of nonwoven or woven fabric having a mean fiber diameter of 0.3 to 1.6 µm. The fibers used are synthetic fibers or regenerated fibers. Fiber materials mentioned are polyamide, polyester, polyacrylonitrile, polytrifluorochloroethylene, polymethyl methacrylate, polystyrene, polyethylene, polypropylene, cellulose and cellulose acetate.

JP 2005/144330 A discloses a blood filter consisting of a copolymer of a polyester or polyketone with a polyalkylene glycol. The filter contains 5 to 90 vol% of pores having an average diameter of 0.001 to 10 µm.

### SUMMARY OF THE INVENTION

An embodiment of this invention is directed to a module to be used in the treatment of diseases which may be caused by activated immune cells.

Another embodiment of this invention is directed to a module to leukoreduce or filter white blood cells from blood and/or blood products.

The module of this invention comprises at least a housing having first and second ends. The housing further comprises a cavity and at least one fluid flow passage to enable blood to flow into the cavity, filtration material within the cavity of the housing and a fluid flow device which extends through the filtration material.

This invention also includes a method of decreasing the amount of leukoctyes in blood by flowing the blood through a module containing cycloolefin copolymer filtration material and allowing at least a portion of the leukocytes contained in the blood to be retained in the material.

### BRIEF DESCRIPTION OF THE DRAWINGS

FIG. 1 is a cross-sectional view of an embodiment of a treatment module of this invention.
FIG. 2 is an exterior view of another embodiment of a treatment module of this invention.
FIG. 3 is a cross-sectional view of the embodiment of the treatment module shown in FIG. 2 taken along axis A.
FIG. 4 is a cross sectional view of the fluid flow device used in the treatment modules of this invention.
FIG. 5 is a cross-sectional view of the treatment module shown in FIG. 2 taken along axis B.
FIG. 6A shows a top-down view of an embodiment of the adsorbent material used with the present invention.
FIG. 6B shows a side view of an embodiment of the adsorbent material used with the present invention.
FIG. 6C shows the cross-sectional view of the distribution channels in the filtration material when the treatment module is assembled.
FIG. 7 is a cross-sectional view of another embodiment of a treatment module.
FIG. 8 schematically illustrates a system for providing treatment to a patient according to one embodiment of the present invention.
FIG. 9 is a graph comparing cell counts of activated v. non-activated white blood cells after passage of blood through the treatment module.
FIG. 10 is a graph showing FACS results after passage of blood through the treatment module of the present invention.
FIG. 11 is a graph showing cytokine production by quiescent white blood cells after removal of activated white blood cells.

### DETAILED DESCRIPTION OF THE INVENTION

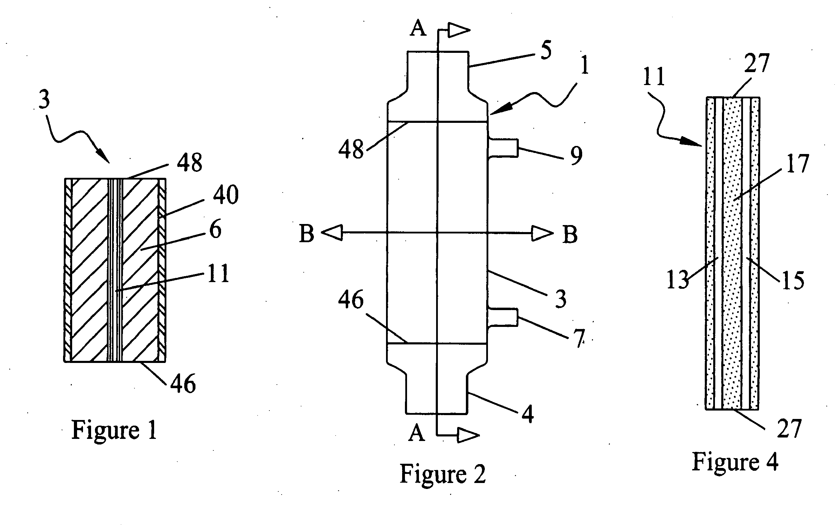

As shown in FIG. 1, the blood treatment module **1** of this invention consists of at least a housing **3,** first **46** and second **48** ends, and filtration media or material **6** contained within the cavity **42** formed by the housing **3.** A fluid flow device **11** (see FIGS. 1, 3 and 4) extends from the first end **46** of the housing through the filtration material **6** to the second end **48.**

It should be noted that in the drawings, common elements are depicted by common numerals.

As shown in FIG. 1, the housing **3** may be a hollow casing having an inside cavity **42.** The cavity **42** has an inside surface **40** which is the inner wall of the housing forming the cavity **42.** The housing also has first **46** and second **48** ends. The design of this treatment module allows for the use of housings or casings **3** which are standard in the commercial dialysis industry. They are easy to obtain, and no special manufacturing or additional design is needed. One example of a standard housing which may be used with this invention is FH22 available from Gambro Dialysatoren, (Hechingen, DE). Although the housing **3** is shown in the drawings to be cylindrical in shape, any housing shape may be used.

In another embodiment shown in FIG. 2, first and second end caps **4, 5** which abut the ends **46, 48** respectively, of the housing **3** may also be used. End caps **4, 5** for the housing **3** are also available from Gambro Dialysatoren, Hechingen, DE. At least the second end cap **5** includes a circular fluid channel **64** which extends the length of the end cap **5,** from the first end **60** of the end cap **5** to the second end **62** of the end cap. The circular fluid channel **64** circumferentially surrounds at least some portion of an end **27** of the fluid flow device **11.** The circular fluid channel **64** of the second end cap **5** helps to direct the treated fluid to flow from the fluid flow device **11** through the second end cap **5** and out of the housing **3.**

End cap **4** seals off the end **46** of the housing **3** to prevent the flow of fluid therethrough. An end cap **4** does not have to be used however. End **46** of housing **3** may be sealed closed by any means known in the art. The housing **3** and end caps **4, 5** are commonly made of polycarbonate, but any polymeric material which is biocompatible may be used. Alternatively, and/or additionally, the housing **3** and end caps **4, 5** may be coated or treated with a material to increase biocompatability. SMA, or polysiloxane copolymer is one such material which may be used.

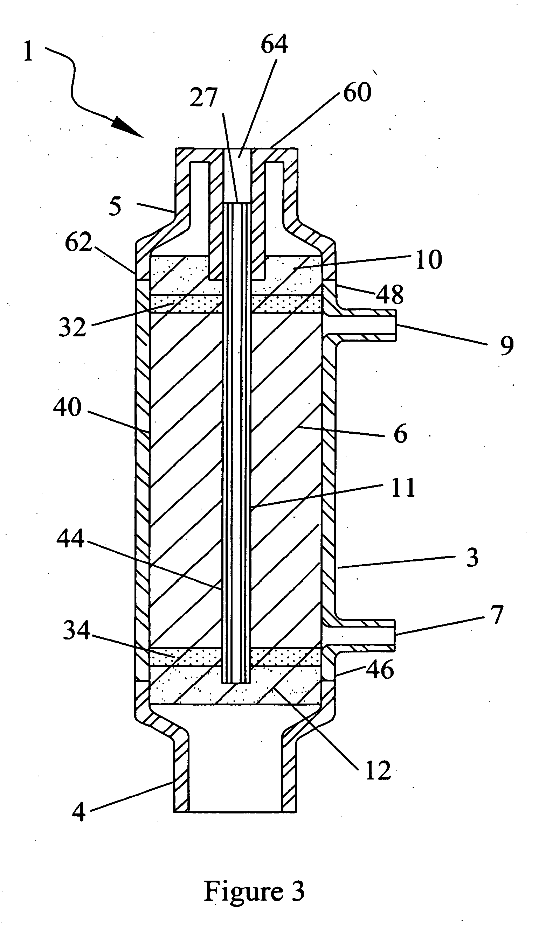

As shown in the embodiments of FIGS. 2 and 3, at least a first fluid passage 7 fluidly extends from outside of the module **3** to the inside surface **40** of the cavity **42** to allow fluid to enter the inside cavity **42** of housing **3.** First fluid passage 7 enables the fluid to be treated to flow into the inside **42** of housing **3.** A second fluid passage **9** enables residual air trapped in the module to flow out of the housing **3.** Although shown as having the first and second fluid passages (**7, 9**) located on the same side of the housing **3,** the fluid passages may be located anywhere on the housing.

In another embodiment, (not shown), the first **7** and second **9** fluid passages may be located near the center of the housing **3,** and the first fluid passage 7 may be located on the housing opposite the second fluid passage **9.**

In one embodiment of the treatment module **1** which is shown in FIG. 3, a first end potting portion **12** and a second end potting portion **10** are respectively provided at both ends of the housing **3** to hold the filtration material **6** and the fluid flow device **11** in place. The first end potting portion **12** also closes off the first end of the cavity **46** to prevent the flow of fluid out of the cavity **42.** In an embodiment, the potting may be polyurethane, however any potting material which is used in the extracorporeal blood circulation industry may be used.

It should be noted that potting material need not be used in this invention. At least the first end of the cavity **46** may be sealed off by any means known in the art, including but not limited to a thixotropic urethane, hot plate welding, compression, shrink wrap, gluing or taping. The filtration material **6** and fluid flow device **11** also may be secured within the cavity **42** of the housing **3** by any means.

The fluid flow device 11 extends through the filtration material 6 from the first end **46** of the housing **3** to the second end **48** of the housing **3** through the cavity **42** of the housing **3.** As discussed above, the fluid flow device **11** fits into the circular fluid channel **64** of end cap **5** enabling treated fluid to flow out of the treatment module **1,** creating a third fluid passage.

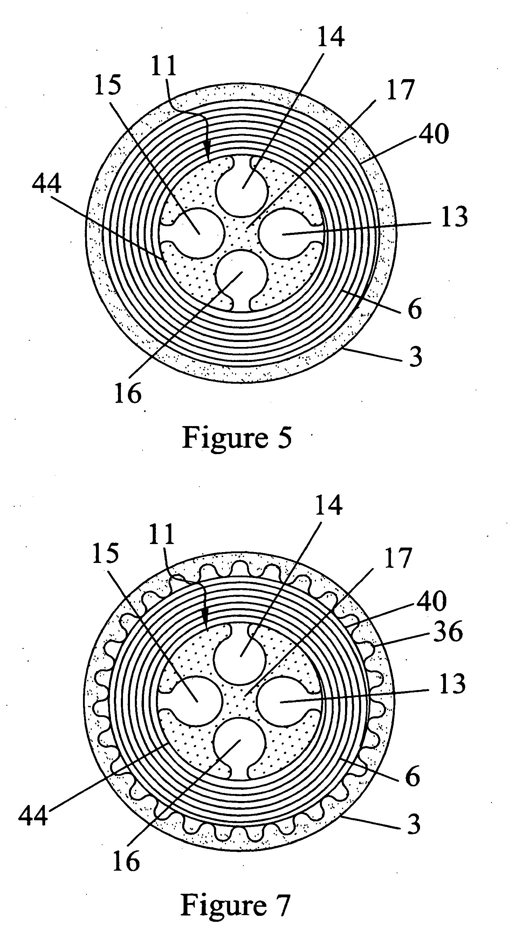

As shown in FIGS. 4 and 5, the fluid flow device **11** has a solid core **17,** and at least one trough (see **13, 14, 15** or **16**) extending longitudinally the length of the device located in the outer circumferential face thereof. In one embodiment, one fluid trough **13** (or **14**) may be located opposite another trough **15** (or **16).** As seen in FIG. 5, there are four troughs **13, 14, 15, 16.** Although four troughs are shown, any number of troughs will achieve the objective of collecting the fluid to be treated which has flowed through the filtration material **6,** and conducting the fluid toward the outside of the treatment module **1.** The more troughs there are in the fluid flow device **11,** the faster the fluid can be collected and flowed out of the treatment module **1.** The troughs may be any size and any shape, although it is important that the troughs have smooth surfaces with no sharp edges. The size and shape of the troughs should be large enough to avoid the possibility of blood clots forming in the fluid troughs.

A fluid flow device **11** such as the one described above is easy and cost effective to manufacture since it can be coextruded in one step. Furthermore, the fluid flow device **11** has a smooth surface, which is desirable since blood can aggregate and form clots in locations of rough sections of fluid flow devices, or can cause lysis of the blood cells, which is highly dangerous to a patient: Compared to the blood flow devices disclosed in United States Patent No. 6659289, no holes need to be drilled into the device to allow blood to pass in and out of the center of the device. Drilling holes not only increases cost of manufacture, but also increases the likelihood of creating sharp or rough edges.

The filtration material **6** is wrapped or wound around the outer circumference **44** of the fluid flow device **11** forming concentric layers or rings as shown in FIG. 5.

The material **6** in the module **1** may be any desired thickness, depending upon the initial thickness of the filtration material itself, and the number of times the material is wrapped around the fluid flow device **11.** The material 6 should be wrapped or wound around the fluid flow device **11** enough times to make a bundle which fits snugly into the cavity **42** of the housing **3.**

In an embodiment shown in FIG. 5, the outer portion of the wrapped filtration material **6** may be adhered to at least a portion of the inside surface **40** of the cavity **42** using heat, radio frequency or other types of welding. Adhering at least a portion of the filtration material **6** to the inside surface **40** of the cavity **42** helps to prevent fluid from flowing along the inside surface **40** of the cavity **42** and out of the third fluid passage without entering the filtration material **6**. In another embodiment, the portion of the filtration material **6** closest to the fluid flow device **11** may be welded to the outside surface **44** of the fluid flow device **11.** As shown in FIG 3, the filtration material **6** is welded to the outside surface **44** of the fluid pipe at two different positions **32** and **34** respectively to force the fluid to be treated to flow through the material.

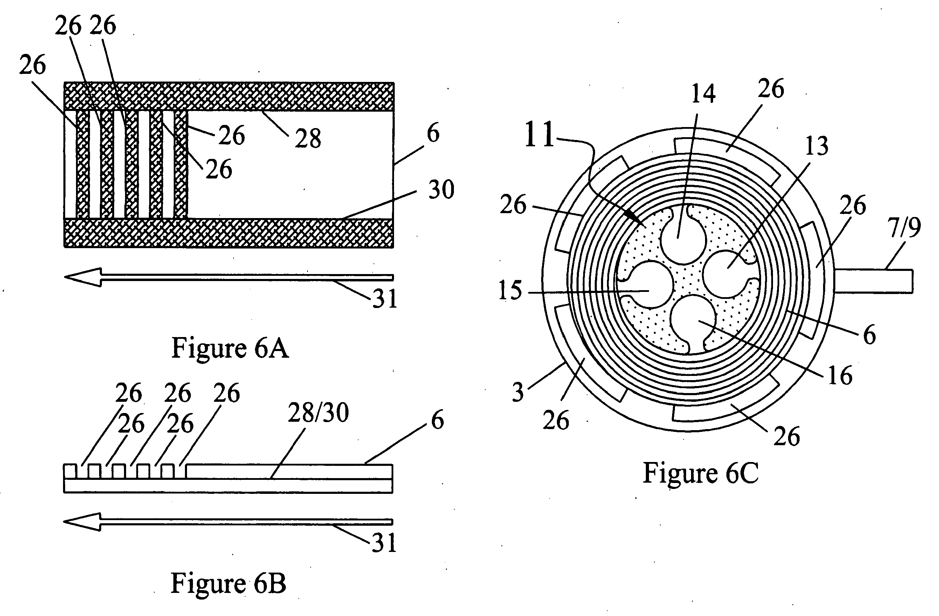

In another embodiment, the wrapped filter material **6** could be constructed in such a way that distribution channels **26** (see Figs. 6A-C) which direct the fluid to be treated to flow in a predetermined flow path through the device are formed between the filter media **6** and the inside of the housing **42.** This may be done by heat treating or welding or sealing layers of the flat filter media together before it is assembled in the housing.

In FIG 6A a top view of the flat sheet filtration material **6** is shown with a series of welded distribution channels **26.** As shown in FIG. 6C, the distribution channels **26** are located on the portion of material closest to and facing the inside surface **40** of the cavity **42** of the housing **3.** To achieve this configuration, the absorbent material **6** is wrapped around the fluid flow device **11** in the direction of the arrow **31,** starting at the end opposite the distribution channels.

The media **6** may also have barrier welds **28, 30** running the length of the media to prevent the potting (if used) from seeping into the filter material. The barrier welds are formed by sealing layers of media together. If potting is not used, no barrier welds are necessary.

FIG. 6B shows a side view of the distribution channels **26** and barrier welds **28/30.** One advantage to the creation of distribution channels **26** on the media is that the channels force the fluid to perfuse equally throughout the device.

In an embodiment shown in cross section in FIG. 7, element **36** represents a sealing material which may be placed along inner surface **40** of the cavity **42.** Instead of sealing welds **32,34,** the sealing material **36** forces the blood to flow radially inward, through the filtration material **6** layer by layer, and away from the inner surface **40** of the cavity **42.**

The filtration material **6** is a web or mat of melt blown polymeric fibers. The polymeric material which is used is a cycloolefin copolymer (or COC) (sold under the tradename Topas, available from Topas Advanced Polymers, GmbH, Germany). This material is further described in expending application WO 2007/025738.

The melt-blown COC material used may be left unmodified or may be modified to change particular characteristics of the material.

For example, the COC material may be modified with ligands or other bioactive substances which may be immobilized on the surface of the fiber to give added specificity to the material. For example, ligands to any of the cytokines described in the background above may be adhered to the surface of the material to selectively remove specific cytokines from the blood stream.

Other substances which modify characteristics of the material such as wettability and biocompatibility may also be used.

Substances which may be used to increase the wettability of the COC material are polysorbate compounds. Tween^{®} 20 or Polysorbate 20 (polyoxyethylene-sorbitane monolaurate) is one such substance. In combination with the COC material, Tween^{®} 20 may help enable better functionality of the material as a leukoreduction filter.

Substances such as polysiloxane co-polymer (SMA) may be used to enhance biocompatibility of the COC material.

The treatment module of this invention may use a combination of filtration materials, for example, a module may be constructed that utilizes a combination of both modified and unmodified filtration material.

To make the treatment module **1,** the filtration material **6** (modified or unmodified) is wrapped around the fluid flow device **11** to make a bundle, and the bundle is inserted into the cavity **42** of housing **3.** The assembled material **6** and fluid flow device **11** are fixed within the cavity **42** of the housing **3,** and the circular fluid channel **64** of end cap **5** is fitted over an end **27** of the fluid flow device **11.** If used, end cap **4** may be assembled on the other end of the cavity **46.** If end cap **4** is not used, end **46** of the module **1** is closed off by other methods. The end caps **4, 5** can be secured by solvent bonding or other known attachment techniques. All of the elements may also be held in place with potting at each end **12, 10** (see FIG. 3), or may be held in place by other known methods.

Blood to be treated by removing the white blood cells enters the housing **3** through first fluid passage 7. Blood flows whether by gravity or with the help of a pump through the filtration material **6** into the troughs **13, 14, 15, 16** of the fluid flow device **11.** Blood flows through the troughs and out of the housing through the third fluid passage **5.**

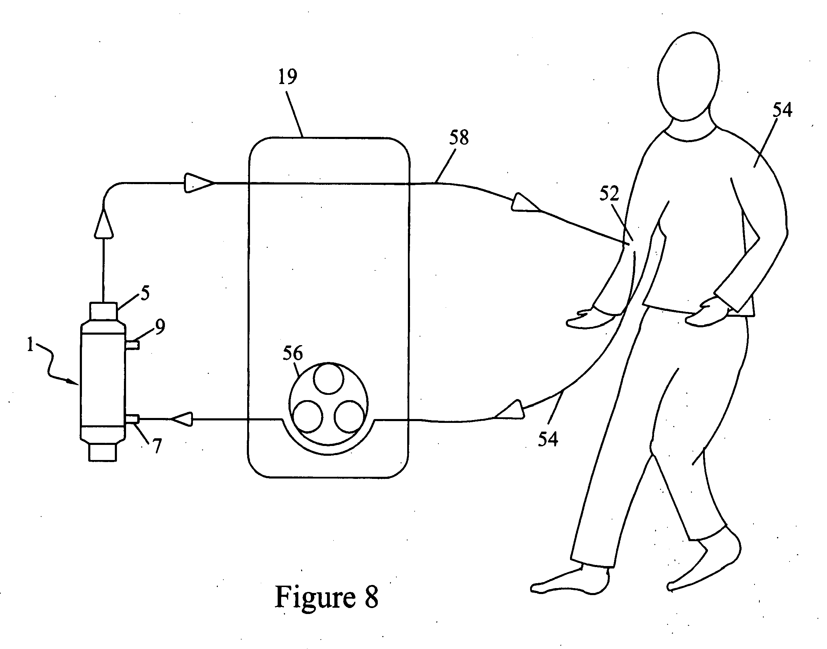

It is also envisioned that the treatment module **1** could be a component of a hemoadsorption system. The blood treatment module could be used with a standard extracorporeal blood processing machine **19** such is shown in FIG. 8. The extracorporeal blood processing machine **19** may be connected to a patient **50** through vascular access **52.** Blood to be treated is pumped out of the patient **50** through conduit **54** and into extracorporeal blood processing machine **19,** by pump **56.** Blood is pumped into treatment module 1 through first fluid passage 7. Blood flows through the filtration material **6,** into fluid flow device **11,** and out of the module **1** through fluid passage **5.** Once air is removed from the system, fluid flow passage **9** is closed or clamped off to prevent treated fluid from flowing out the module **1** through fluid flow passage **9.** Treated blood flows back to the patient through conduit **58.** Although only one pump is shown in FIG. 8, it should be noted that any number of pumps can be used.

The extracorporeal blood processing machine **19** may be a dialysis machine, and the treatment module **1** may be used to remove white blood cells from whole blood during the dialysis procedure. The module **1** may also be used as a component of other treatment procedures such as those that remove various toxins from blood. The treatment module **1** may also be used with a machine to separate whole blood into components. The treatment module 1 may be used to remove white blood cells from any of the separated blood components, either concurrently with the separation procedure, or at any point after the separation procedure is completed and the blood components are separated.

During passage of blood through the treatment module **1,** leukocytes are trapped by the fibers and/or adhere to the fibers of the material **6** and are removed from the blood. Preferential removal of activated leukocytes as compared to removal of all leukocytes may help to prevent sepsis or other diseases, because, as discussed above, non-activated leukocytes do not release cytokines, and should not additionally contribute to the development of sepsis. If activated leukocytes are removed, those leukocytes which are not activated will still be available in the blood to mount an immune response should one become necessary.

### Examples

The following examples help illustrate the use of the module in the treatment of sepsis.

### Example 1

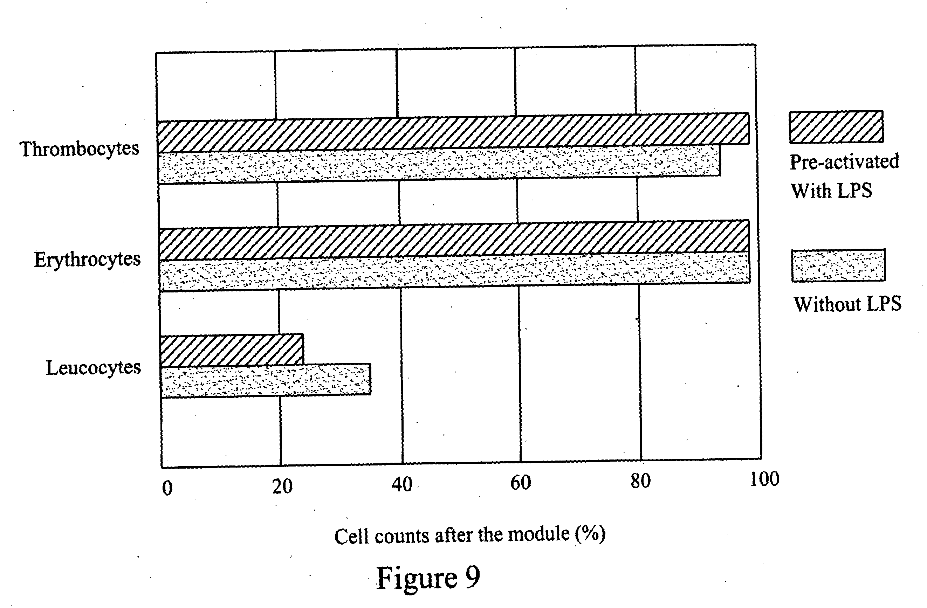

500 mL of donated human blood was split into two samples, each containing 250 mL of whole blood. The blood was continuously and slowly stirred at 37 °C to prevent clotting. A representative sample from each flask was removed to determine the number of white blood cells originally present in each of the 250 mL whole blood samples. 1 U/mL lipopolysaccharide (LPS), to act as an endotoxin activator of white blood cells, was then added to one of the flasks (hereafter called the activated blood flask) to activate the white blood cells contained in the whole blood. No LPS was added to the whole blood in the other flask (hereafter called the control blood flask). Both the control blood and activated blood flasks were incubated with stirring at 37 °C for four hours. Both the activated and control blood was continuously pumped through treatment modules containing COC filtration material at a rate of 50 mL/min. Blood samples were removed every two minutes from blood which had flowed through the filter and the number of activated white blood cells remaining in the circulating blood were manually counted. The results are shown in FIG. 9.

As can be seen, the COC filtration material appears to remove both activated and non-activated leukocytes within 20 minutes of continuous flow through the treatment module. It also appears that activated leukocytes (pre-activated with LPS) are preferentially removed.

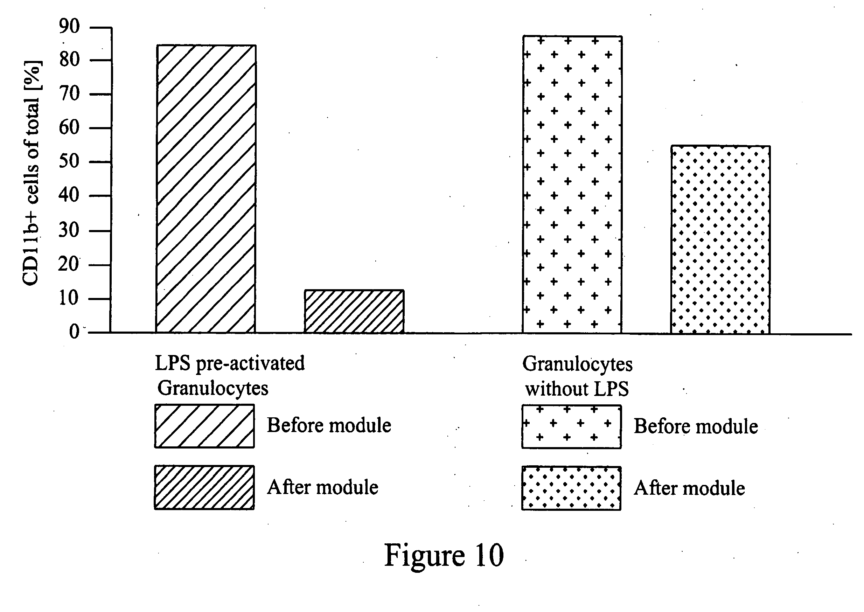

This behavior was further confirmed by cell staining. Whole blood was initially stained with fluorescent markers for CD11b, which is a marker of monocyte and granulocyte activation. CD11b fluorescent antibody is available from Becton, Dickinson & Co. (Franklin Lakes, NJ, USA). Samples from the above-described setup were removed every 2 minutes, and the percentage of activated v. non-activated leukocytes remaining in the circulating blood was determined using FACS analysis. The results are shown in FIG. 10. As the results in FIG. 9 indicate, it appears that activated granulocytes are preferentially removed by the COC filtration material.

### Example 2

As described above, it is known that LPS binds to receptors on proinflammatory cells, causing a cascade of events beginning with granulocyte and monocyte activation and ending with cytokine release. Cytokines not only increase activation of cytokine-releasing cells, but also increase the amount of cytokine receptors put on the cell's surface.

If activated granuloyctes are eliminated from circulation, the inventors hypothesized that the amount of LPS produced by the activated cells would also be reduced, since LPS binds to granulocytes, causing their activation, which causes cytokine release by the activated granuloytes. The cytokines bind to granulocytes causing further granulocyte activation and production of a hyperreactive state. By removing the cells which have LPS bound to them, the cycle leading to sepsis may be broken.

To investigate whether LPS is actually depleted through removal of activated cells, whole blood was incubated with 0 (hereafter control blood); or 0.3, 1, 3, 10 or 30 IU/mL LPS (hereafter called activated blood). The control and activated blood were incubated between 30-90 minutes at room temperature with stirring.

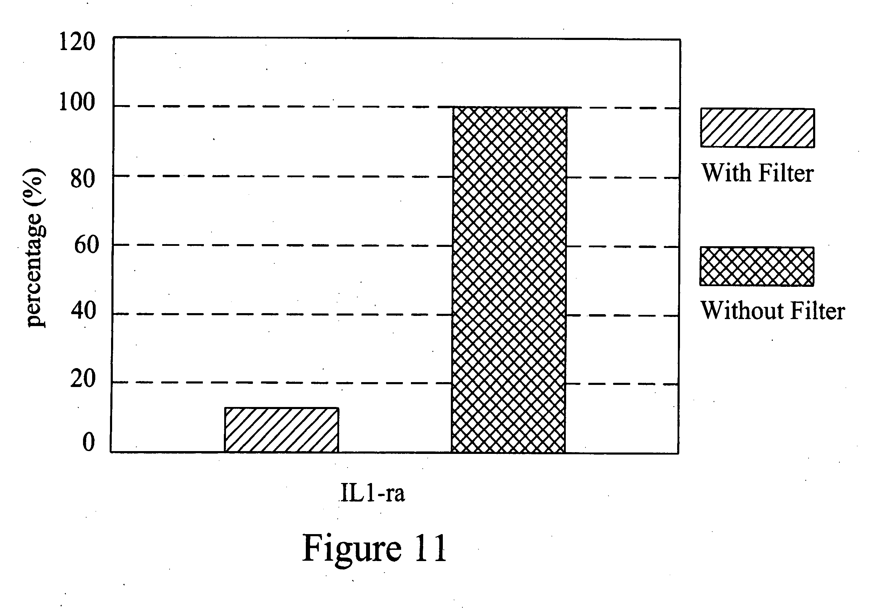

After incubation, the activated blood was filtered through a standard commercially available leukoreduction filter to remove the white blood cells present. 500 µL samples of the leukoreduced activated blood were plated in a 24-well plate. Activated whole blood which was not filtered was also plated. Freshly separated quiescent white blood cells from the same donor were added to both the filtered and non-filtered blood in the wells. After a six hour incubation at 37 °C in 5% CO₂ atmosphere, the cytokine IL-1ra was assayed using a conventional ELISA assay. As shown by the graph in FIG. 11, IL-1ra is present to a much greater extent in the wells containing activated cells which were not filtered, compared to whole blood which was filtered. Such results appear to suggest that removal of activated leukocytes does contribute to decreased cytokine production.

## Claims

1. A module (1) to be used in the treatment of a fluid comprising blood or blood components comprising:
a housing (**3**) comprising first (**46**) and second (**48**) ends;
a cavity (**42**) defined by the housing (**3**) between the first (**46**) and second (**48**) ends;
at least one fluid flow passage (**7**) to enable blood to flow into the cavity (**42**);
a filtration material (**6**) in the cavity (**42**) of the housing (**3**);
**characterized in that**
the module comprises a fluid flow device (11) comprising:
a solid core (**17**) having
at least one trough (**13, 14, 15, 16**) for collecting the fluid which has flowed through the filtration material (**6**) and conducting the fluid towards the outside of the module (**1**), said at least one trough (**13, 14, 15, 16**) having a smooth surface with no sharp edges, extending longitudinally the length of the fluid flow device (**11**);
and having at least one end (**27**) and which extends through the filtration material (**6**); and
the filtration material (**6**) comprises a polymeric melt-blown web comprising cycloolefin copolymer.

2. The module of claim 1 wherein the housing (**3**) further comprises a second fluid flow passage (**9**).

3. The module of claim 2 wherein the second fluid flow passage (**9**) communicates with the cavity (**42**) to permit air to flow out of the housing (**3**).

4. The module of claim 1 further comprising an end cap (**5**) for closing off at least a portion of one end (**48**) of the housing (**3**),
wherein the end cap (**5**) further comprises a circular fluid channel (**64**).

5. The module of claim 1 further comprising a second end cap (**4**) for closing off the other end (**46**) of the housing (**3**).

6. The module of claim 4 wherein the circular fluid channel (**64**) of the end cap (**5**) surrounds at least a portion of the at least one end (**27**) of the fluid flow device (**11**) to enable fluid to flow out of the cavity (**42**).

7. The module of claim 1 wherein the other end (**46**) of the cavity (**42**) is closed off to prevent the flow of fluid there through.

8. The module of claim 1 wherein the filtration material (**6**) selectively removes activated leukocytes from the blood or blood components.

9. The module of claim 1 wherein the filtration material (**6**) removes leukocytes from the blood or blood components.

10. The module of claim 1 wherein the filtration material (**6**) is modified with a material to increase wettability.

11. The module of claim 1 wherein the filtration material (**6**) is modified with a material to increase biocompatibility.

12. The module of claim 11 wherein the filtration material (**6**) is further comprised of cyclic olefin copolymer material modified with SMA.

13. The module of claim 10 wherein the filtration material (**6**) is further comprised of cyclic olefin copolymer material modified with Tween^{®} 20.

14. The module of claim 1 wherein the housing cavity (**42**) comprises an inside surface portion (**40**) and wherein the filtration material (**6**) is adhered to at least a portion of the inside surface (**40**) of the cavity (**42**)**.**

15. The module of claim 1 wherein the filtration material (**6**) is further comprised of fluid distribution channels (**26**).

16. The module of claim 1 wherein the filtration material (6) is further comprised of barrier welds (**28, 30**).

17. The module of claim 1 wherein the fluid flow device (**11**) further comprises a plurality of troughs (**13, 14, 15, 16**).

18. The module of claim 1 wherein the filtration material (**6**) and fluid flow device (**11**) are sealed in place within the cavity (**42**) of the housing (**3**).

19. The module of claim 1 wherein the filtration material (**6**) is wrapped circumferentially around the fluid flow device (**11**).

20. The module of claim 1 wherein the filtration material (**6**) further comprises a combination of modified and unmodified material.

21. A method of making a module for removing white blood cells from blood or blood components comprising the steps of:
wrapping filtration material (**6**) circumferentially around a fluid flow device (**11**);
inserting the assembled filtration material (**6**) and fluid flow device (11) into a cavity (**42**) of a housing (**3**) having first (**46**) and second (**48**) ends, and at least one fluid flow passage (7); and
sealing the assembled filtration material (**6**) and fluid flow device (11) into the cavity (**42**) of the housing (**3**), **characterized in that**
the fluid flow device (**11**) comprises a solid core (17); and at least one trough (**13, 14, 15, 16**) having a smooth surface with no sharp edges, extending longitudinally the length of the fluid flow device (**11**); and
the filtration material (**6**) comprises a polymeric melt-blown web comprising cycloolefin copolymer.

22. The method of claim 21 further comprising installing a first end cap (**4**) and a second end cap (**5**) onto the first (**46**) and second (**48**) ends of the housing.

23. The method of claim 22 wherein the module further comprises a second fluid flow passage (**9**).

24. The method of making the module of claim 23 further comprising coextruding the fluid flow device (**11**).

25. A method of decreasing the amount of leukocytes in blood or a blood product ex vivo comprising the steps of:
flowing the blood or blood product through a module containing cycloolefin copolymer filtration material according to any one of claims 1 to 20; and
allowing at least a portion of leukocytes contained in the blood or blood product to be retained in the material.

26. The method of claim 25 further comprising reducing the number of activated leukocytes.

27. The method of claim 26 wherein the step of reducing the number of activated leukocytes in the blood or blood product further comprises reducing the amount of cytokines in the blood.

## Patentansprüche

1. Modul **(1)** zur Verwendung in der Behandlung einer Blut oder Blutbestandteile enthaltenden Flüssigkeit, umfassend:
ein Gehäuse **(3)** mit erstem **(46)** und zweitem **(48)** Ende;
einen durch das Gehäuse **(3)** zwischen dem ersten **(46)** und zweiten **(48)** Ende umgrenzten Hohlraum **(42);**
mindestens einen Flüssigkeitsstromkanal **(7),** der den Zufluss von Blut in den Hohlraum **(42)** ermöglicht;
ein Filtermaterial **(6)** in dem Hohlraum **(42)** des Gehäuses **(3);**
**gekennzeichnet dadurch, dass**
das Modul eine Flüssigkeitsleitvorrichtung **(11)** aufweist, die
einen festen Kern **(17)** mit
mindestens einer Mulde **(13, 14, 15, 16)** zur Sammlung der Flüssigkeit, die durch das Filtermaterial **(6)** geflossen ist und Führung der Flüssigkeit in Richtung der Außenseite des Moduls **(1),** wobei die mindestens eine Mulde **(13, 14, 15, 16)** eine glatte Oberfläche ohne scharfe Kanten aufweist und sich in Längsrichtung über die Länge der Flüssigkeitsleitvorrichtung **(11)** erstreckt, umfasst; und
mindestens ein Ende **(27)** aufweist und sich durch das Filtermaterial **(6)** erstreckt; und
das Filtermaterial **(6)** ein Cycloolefin-Copolymer enthaltendes polymeres schmelzgeblasenes Vlies umfasst.

2. Modul gemäß Anspruch 1, worin das Gehäuse **(3)** zusätzlich einen zweiten Flüssigkeitsstromkanal **(9)** enthält.

3. Modul gemäß Anspruch 2, worin der zweite Flüssigkeitsstromkanal **(9)** mit dem Hohlraum **(42)** verbunden ist, um das Entweichen von Luft aus dem Gehäuse **(3)** zu ermöglichen.

4. Modul gemäß Anspruch 1, das zusätzlich eine Endkappe **(5)** umfasst, um mindestens einen Teil eines Endes **(48)** des Gehäuses **(3)** zu verschließen,
wobei die Endkappe **(5)** weiterhin einen kreisförmigen Flüssigkeitskanal **(64)** aufweist.

5. Modul gemäß Anspruch 1, das zusätzlich eine zweite Endkappe **(4)** um das andere Ende **(46)** des Gehäuses **(3)** zu verschließen.

6. Modul gemäß Anspruch 4, bei dem der kreisförmige Flüssigkeitskanal **(64)** der Endkappe **(5)** mindestens einen Teil des mindestens einen Endes **(27)** der Flüssigkeitsleitvorrichtung **(11)** umgibt, um das Abfließen von Flüssigkeit aus dem Hohlraum **(42)** zu ermöglichen.

7. Modul gemäß Anspruch 1, bei dem das andere Ende **(46)** des Hohlraums **(42)** verschlossen ist, um den Durchfluss von Flüssigkeit durch dieses zu verhindern.

8. Modul gemäß Anspruch 1, bei dem das Filtermaterial **(6)** aktivierte Leukozyten aus dem Blut oder den Blutbestandteilen selektiv entfernt.

9. Modul gemäß Anspruch 1, bei dem das Filtermaterial **(6)** Leukozyten aus dem Blut oder den Blutbestandteilen entfernt.

10. Modul gemäß Anspruch 1, bei dem das Filtermaterial **(6)** mit einem Material modifiziert ist, um die Benetzbarkeit zu erhöhen.

11. Modul gemäß Anspruch 1, bei dem das Filtermaterial **(6)** mit einem Material modifiziert ist, um die Biokompatibilität zu erhöhen.

12. Modul gemäß Anspruch 11, bei dem das Filtermaterial **(6)** außerdem mit SMA modifiziertes Cycloolefin-Copolymermaterial umfasst.

13. Modul gemäß Anspruch 10, bei dem das Filtermaterial **(6)** außerdem mit Tween^{®} 20 modifiziertes Cycloolefin-Copolymermaterial umfasst.

14. Modul gemäß Anspruch 1, bei dem der Hohlraum **(42)** des Gehäuses einen Anteil einer Innenoberfläche **(40)** umfasst und worin das Filtermaterial **(6)** an mindestens einem Teil der Innenoberfläche **(40)** des Hohlraums **(42)** befestigt ist.

15. Modul gemäß Anspruch 1, bei dem das Filtermaterial **(6)** außerdem Flüssigkeitsverteilerkanäle **(26)** umfasst.

16. Modul gemäß Anspruch 1, bei dem das Filtermaterial **(6)** außerdem Sperrnähte **(28, 30)** umfasst.

17. Modul gemäß Anspruch 1, bei dem die Flüssigkeitsleitvorrichtung **(11)** außerdem mehrere Mulden **(13, 14, 15, 16)** aufweist.

18. Modul gemäß Anspruch 1, bei dem das Filtermaterial **(6)** und die Flüssigkeitsleitvorrichtung **(11)** innerhalb des Hohlraums **(42)** des Gehäuses **(3)** fest angebracht sind.

19. Modul gemäß Anspruch 1, bei dem das Filtermaterial **(6)** um die Flüssigkeitsleitvorrichtung **(11)** herum gewickelt ist.

20. Modul gemäß Anspruch 1, bei dem das Filtermaterial **(6)** außerdem eine Kombination von modifiziertem und nicht modifiziertem Material enthält.

21. Verfahren zur Herstellung eines Moduls zur Entfernung weißer Blutzellen aus Blut oder Blutbestandteilen, umfassend die Schritte:
Wickeln von Filtermaterial **(6)** um eine Flüssigkeitsleitvorrichtung **(11)** herum;
Einsetzen des montierten Filtermaterials **(6)** und der Flüssigkeitsleitvorrichtung **(11)** in einen Hohlraum **(42)** eines Gehäuses **(3)** mit ersten **(46)** und zweiten **(48)** Enden und mindestens einem Flüssigkeitsstromkanal **(7);** und
Befestigen des montierten Filtermaterials **(6)** und der Flüssigkeitsleitvorrichtung **(11)** in dem Hohlraum **(42)** des Gehäuses **(3),**
**dadurch gekennzeichnet, dass**
die Flüssigkeitsleitvorrichtung **(11)** einen festen Kern **(17)** und mindestens eine Mulde **(13, 14, 15, 16)** mit einer glatten Oberfläche ohne scharfe Kanten, die sich entlang der Längsrichtung über die Länge der Flüssigkeitsleitvorrichtung **(11)** erstrecht, aufweist; und
das Filtermaterial **(6)** ein Cycloolefin-Copolymer enthaltendes polymeres schmelzgeblasenes Vlies umfasst.

22. Verfahren gemäß Anspruch 21, außerdem umfassend das Anbringen einer ersten Endkappe **(4)** und einer zweiten Endkappe **(5)** auf dem ersten **(46)** und zweiten **(48)** Ende des Gehäuses.

23. Verfahren gemäß Anspruch 22, bei dem das Modul außerdem einen zweiten Flüssigkeitsstromkanal **(9)** aufweist.

24. Verfahren zur Herstellung des Moduls gemäß Anspruch 23, das außerdem die Coextrusion der Flüssigkeitsleitvorrichtung **(11)** umfasst.

25. Verfahren zur Verringerung der Anzahl von Leukozyten in Blut oder einem Blutprodukt *ex vivo,* umfassend die Schritte:
Durchleiten des Bluts oder Blutprodukts durch ein Cycloolefin-Copolymer Filtermaterial enthaltendes Modul gemäß einem der Ansprüche 1 bis 20; und
Zulassen, dass mindestens ein Teil der im Blut oder Blutprodukt enthaltenen Leukozyten in dem Material zurückgehalten wird.

26. Verfahren gemäß Anspruch 25, das außerdem die Verringerung der Anzahl aktivierter Leukozyten umfasst.

27. Verfahren gemäß Anspruch 26, bei dem der Schritt der Verringerung der Anzahl aktivierter Leukozyten im Blut oder Blutprodukt außerdem die Verringerung der Menge von Cytokinen im Blut umfasst.

## Revendications

1. Module (1) à utiliser dans le traitement d'un fluide comprenant le sang ou des composants du sang, comprenant :
un boîtier (3) comprenant une première (46) et une seconde (48) extrémité ;
une cavité (42) définie par le boîtier (3) entre la première (46) et la seconde (48) extrémité ;
au moins un passage d'écoulement de fluide (7) pour permettre au sang de s'écouler dans la cavité (42) ;
un matériau de filtration (6) dans la cavité (42) du boîtier (3) ;
**caractérisé en ce que**
le module comprend un dispositif d'écoulement de fluide (11) comprenant
un noyau plein (17) ayant
au moins une goulotte (13, 14, 15, 16) pour collecter le fluide qui s'est écoulé à travers le matériau de filtration (6) et pour conduire le fluide vers l'extérieur du module (1), ladite au moins une goulotte (13, 14, 15, 16) ayant une surface lisse dépourvue de bords vifs, s'étendant longitudinalement sur la longueur du dispositif d'écoulement de fluide (11) ;
et ayant au moins une extrémité (27) et qui s'étend à travers le matériau de filtration (6) ; et
le matériau de filtration (6) comprend une nappe en polymère réalisée par extrusion-soufflage comprenant un copolymère de cyclooléfine.

2. Module selon la revendication 1, dans lequel le boîtier (3) comprend en outre un second passage d'écoulement de fluide (9).

3. Module selon la revendication 2, dans lequel le second passage d'écoulement de fluide (9) communique avec la cavité (42) pour permettre à l'air de s'écouler hors du boîtier (3).

4. Module selon la revendication 1, comprenant en outre un capuchon terminal (5) pour refermer au moins une portion d'une extrémité (48) du boîtier (3),
dans lequel le capuchon terminal (5) comprend en outre un canal à fluide circulaire (64).

5. Module selon la revendication 1, comprenant en outre un second capuchon terminal (4) pour refermer l'autre extrémité (46) du boîtier (3).

6. Module selon la revendication 4, dans lequel le canal à fluide circulaire (64) du capuchon terminal (5) entoure au moins une portion de ladite au moins une extrémité (27) du dispositif d'écoulement de fluide (11) pour permettre au fluide de s'écouler hors de la cavité (42).

7. Module selon la revendication 1, dans lequel l'autre extrémité (46) de la cavité (42) est refermée pour empêcher l'écoulement de fluide à travers elle-même.

8. Module selon la revendication 1, dans lequel le matériau de filtration (6) supprime sélectivement des leucocytes activés hors du sang ou des composants sanguins.

9. Module selon la revendication 1, dans lequel le matériau de filtration (6) supprime les leucocytes hors du sang ou des composants sanguins.

10. Module selon la revendication 1, dans lequel le matériau de filtration (6) est modifié avec un matériau pour augmenter sa mouillabilité.

11. Module selon la revendication 1, dans lequel le matériau de filtration (6) est modifié avec un matériau pour augmenter sa biocompatibilité.

12. Module selon la revendication 11, dans lequel le matériau de filtration (6) comprend en outre un matériau copolymère de cyclooléfine modifié avec du SMA.

13. Module selon la revendication 10, dans lequel le matériau de filtration (6) comprend en outre un matériau copolymère de cyclooléfine modifié avec du Tween^{®} 20.

14. Module selon la revendication 1, dans lequel la cavité (42) du boîtier comprend une portion de surface intérieure (40), et dans lequel le matériau de filtration (6) adhère à au moins une portion de la surface intérieure (40) de la cavité (42).

15. Module selon la revendication 1, dans lequel le matériau de filtration (6) comprend en outre des canaux de distribution de fluide (26).

16. Module selon la revendication 1, dans lequel le matériau de filtration (6) comprend en outre des soudures de barrière (28, 30).

17. Module selon la revendication 1, dans lequel le dispositif d'écoulement de fluide (11) comprend en outre une pluralité de goulottes (13, 14, 15, 16).

18. Module selon la revendication 1, dans lequel le matériau de filtration (6) et le dispositif d'écoulement de fluide (11) sont scellés en place à l'intérieur de la cavité (42) du boîtier (3).

19. Module selon la revendication 1, dans lequel le matériau de filtration (6) est enroulé circonférentiellement autour du dispositif d'écoulement de fluide (11).

20. Module selon la revendication 1, dans lequel le matériau de filtration (6) comprend en outre une combinaison de matériau modifié et de matériau non modifié.

21. Procédé pour réaliser un module pour supprimer des globules blancs du sang hors du sang ou de composants sanguins, comprenant les étapes consistant à :
enrouler un matériau de filtration (6) circonférentiellement autour d'un dispositif d'écoulement de fluide (11) ;
insérer le matériau de filtration assemblé (6) et le dispositif d'écoulement de fluide (11) dans une cavité (42) d'un boîtier (3) ayant une première (46) et une seconde (48) extrémité, et au moins un passage d'écoulement de fluide (7) ; et
sceller le matériau de filtration assemblée (6) et le dispositif d'écoulement de fluide (11) dans la cavité (42) du boîtier (3),
**caractérisé en ce que**
le dispositif d'écoulement de fluide (11) comprend un noyau plein (17) ; et au moins de une goulotte (13, 14, 15, 16) ayant une surface lisse dépourvue de bords vifs, s'étendant longitudinalement sur la longueur du dispositif d'écoulement de fluide (11) ; et
le matériau de filtration (6) comprend une nappe polymère, réalisée par extrusion-soufflage, comprenant un copolymère de cyclooléfine.

22. Procédé selon la revendication 21, comprenant en outre l'opération consistant à installer un premier capuchon terminal (4) et un second capuchon terminal (5) sur la première (46) et la seconde (48) extrémité du boîtier.

23. Procédé selon la revendication 22, dans lequel le module comprend en outre un second passage d'écoulement de fluide (9).

24. Procédé pour réaliser le module selon la revendication 23, comprenant en outre de coextruder le dispositif d'écoulement de fluide (11).

25. Procédé pour réduire la quantité de leucocytes dans le sang ou dans un produit sanguin ex vivo, comprenant les étapes consistant à :
amener le sang ou le produit sanguin à s'écouler à travers un module contenant un matériau de filtration en copolymère de cyclooléfine selon l'une quelconque des revendications 1 à 20 ; et
permettre à au moins une portion des leucocytes contenus dans le sang ou dans le produit sanguin d'être retenus dans le matériau.

26. Procédé selon la revendication 25, comprenant en outre l'opération consistant à réduire le nombre de leucocytes activés.

27. Procédé selon la revendication 26, dans lequel l'étape consistant à réduire le nombre de leucocytes activés dans le sang ou dans le produit sanguin comprend en outre de réduire la quantité de cytokines dans le sang.
